**Europäisches Patentamt**

**European Patent Office**

**Office européen des brevets**

(19)

(11) Veröffentlichungsnummer: **0 214 568**
**B1**

(12) EUROPÄISCHE PATENTSCHRIFT

(45) Veröffentlichungstag der Patentschrift:
25.04.90

(21) Anmeldenummer: 86111968.3

(22) Anmeldetag: 29.08.86

(51) Int. Cl.⁴: **B01F 17/00**, A61K 7/00,
A61K 9/10

(54) Öl-in-Wasser-Emulsion.

(30) Priorität: 07.09.85 DE 3531971

(43) Veröffentlichungstag der Anmeldung:
18.03.87 Patentblatt 87/12

(45) Bekanntmachung des Hinweises auf die Patenterteilung:
25.04.90 Patentblatt 90/17

(84) Benannte Vertragsstaaten:
AT BE CH DE FR GB IT LI NL SE

(56) Entgegenhaltungen:
EP-A- 0 111 895
DE-B- 1 948 800
FR-A- 907 881
GB-A- 780 801
US-A- 145 602
US-A- 2 207 256

JOURNAL OF ORGANIC CHEMISTRY, Band 10, 1945,
Seiten 170-174, The Williams & Wilkins Company,
Baltimore, US; A.W. RALSTON et al.: "Solubilities of
binary mixtures of the saturated fatty acids"
SEIFEN-ÖLE-FETTE-WACHSE, Band 109, Nr. 8, 11.
Mai 1983, Seiten 225-229, Augsburg, DE; U. PLOOG:
"Ungesättigte Fettalkohole und ihre Derivate als
Inhaltsstoffe von kosmetischen Mitteln"
FETTE, SEIFEN, ANSTRICHMITTEL, Band 87, Nr. 10,

(73) Patentinhaber: Henkel Kommanditgesellschaft auf
Aktien, Postfach 1100 Henkelstrasse 67,
D-4000 Düsseldorf-Holthausen(DE)

(72) Erfinder: Ansmann, Achim, Dr., Fichtestrasse 19,
D-4010 Hilden(DE)

(74) Vertreter: von Kreisler, Alek, Dipl.-Chem. et al,
Patentanwälte Von Kreisler-Selting-Werner
Deichmannhaus am Hauptbahnhof, D-5000 Köln 1(DE)

(56) Entgegenhaltungen: (Fortsetzung)
Oktober 1985, Seiten 403-408, Industrieverlag von
Hernhaussen KG, Leinfelden-Echterdingen, DE; U.
ZEIDLER et al.: "Über das Spreiten von Lipiden auf der
Haut"
FETTE, SEIFEN, ANSTRICHMITTEL, Band 87, Nr. 10,
Oktober 1985, Seiten 408-410, Industrieverlag von
Hernhausen KG, Leinfelden-Echterdingen, DE; A.
ANSMANN et al.: "Skin-Lotion für nasse Haut - ein
neues Produktrezept"

## Beschreibung

Gegenstand der Erfindung sind Öl-in-Wasser-Emulsionen (im Folgenden O/W-Emulsionen), insbesondere topische, kosmetische und pharmazeutische Zubereitungen in Form einer O/W-Emulsion.

Die Stabilität, Feinteiligkeit und das rheologische Verhalten von O/W-Emulsionen werden in entscheidender Weise durch die Art und Zusammensetzung des Emulgators bestimmt. Unter den Emulgatoren, die für die Herstellung von O/W-Emulsionen eingesetzt werden, kommt den Fettsäureseifen wegen deren hoher Emulgierleistung für alle geläufigen flüssigen und halbfesten Öl- und Fettkomponenten und der besonderen Preiswürdigkeit eine besondere Bedeutung zu. Unter den Fettsäureseifen eignen sich aufgrund ihrer Hydrophilie am besten die Seifen technischer Stearinsäuren. Auf dem Gebiet der Kosmetik spricht man bei solchen Emulsionen auf Stearat-Basis auch von sogenannten Stearat-Cremes.

Die als Emulgatoren meist verwendeten technischen Stearinsäuren sind Mischungen aus Stearinsäure und Palmitinsäure, die durch Verseifung von Talg und Abtrennung der festen Fettsäurefraktion, des sogenannten Stearins, gewonnen werden. Die auf diese Weise gewonnenen "dreifach gepreßten" Stearine bestehen aus 50 bis 55 Gew.-% Palmitinsäure, 40 bis 45 Gew.-% Stearinsäure und geringen Beimengungen an Myristinsäure, Pentadecansäure, Heptadecansäure und Ölsäure. Eine andere technische Stearinqualität wird durch Härtung, d. h. durch Hydrierung der ungesättigten Anteile der Talgfettsäure gewonnen. Ein auf diese Weise gewonnenes Stearin besteht aus 25 bis 30 Gew.-% Palmitinsäure, 60 bis 65 Gew.-% Stearinsäure und geringen Beimengungen an Myristinsäure, Pentadecansäure, Heptadecansäure, Ölsäure und Arachinsäure.

Aus EP-A 111 895 war auch bereits eine Hautpflegeemulsion bekannt, die 1–7 Gew.-% einer Alkaliseife einer Fettsäure mit 12–18 C-Atomen als Emulgator enthält; die als Beispiel 1–4 aufgeführten Duschlotionen enthalten Seifen eines Palmitinsäure-Stearinsäure-Gemisches mit 50 Gew.-% Palmitinsäure und 50 Gew.-% Stearinsäure.

Obwohl über den Einfluß des $C_{16}/C_{18}$-Verhältnisses von Stearinsäure-Palmitinsäure-Gemischen auf das Kristallisationsverhalten und die physikalisch-chemischen Eigenschaften solcher Gemische Untersuchungen angestellt worden sind, z.B. von Tillotson (J. Soc. Cosmet. Chem. 6, (1955), Seiten 40 bis 49), ist über den Einfluß der C-Kettenverteilung dieser Fettsäuren auf das Emulgiervermögen der daraus herstellbaren Seifen nichts bekannt.

O/W-Emulsionen sind metastabile Systeme, bestehend aus einer diskontinuierlichen (inneren) Ölphase und einer kontinuierlichen (äußeren) Wasserphase, die durch die grenzflächenstabilisierende Wirkung des Emulgators für endliche Zeit stabilisiert sind. Bei langdauernder Lagerung, insbesondere bei erhöhter Temperatur, neigen solche Systeme zur Koaleszenz der Öltröpfchen und zur Separation der Phasen. Es bestand daher die Aufgabe, Emulgatoren zu finden, die eine möglichst feinteilige Dispersion der Ölphase in der Wasserphase bewirken und die Emulsion für einen langen Zeitraum stabilisieren.

Es wurde nun überraschend gefunden, daß das $C_{16}/C_{18}$-Verhältnis von Palmitinsäure-Stearinsäure-Gemischen einen signifikanten Einfluß auf das Emulgiervermögen der Seifen solcher Fettsäuregemische hat und daß sich Seifen eines Palmitinsäure-Stearinsäure-Gemisches aus 60 bis 80 Gew.-% Palmitinsäure und 20 bis 40 Gew.-% Stearinsäure besonders gut zur Herstellung von O/W-Emulsionen eignen.

Gegenstand der Erfindung ist daher eine Öl-in-Wasser-Emulsion, bestehend aus einer kontinuierlichen wäßrigen Phase und einer diskontinuierlichen Ölphase, mit einem Emulgator, der ganz oder überwiegend aus der Seife eines Palmitinsäure-Stearinsäure- Gemisches besteht, wobei das Palmitinsäure-Stearinsäure-Gemisch 60 bis 80 Gew.-% Palmitinsäure und 20 bis 40 Gew.-% Stearinsäure enthält. Besonders bevorzugt sind erfindungsgemäße O/W-Emulsionen, in welchen das Palmitinsäure-Stearinsäure-Gemisch aus ca. 70 Gew.-% Palmitinsäure und ca. 30 Gew.-% Stearinsäure besteht.

Das erfindungsgemäß zu verwendende Palmitinsäure-Stearinsäure-Gemisch sollte zwar im wesentlichen aus Palmitinsäure und Stearinsäure bestehen, es ist jedoch für die erfindungsgemäße Eignung nicht abträglich, wenn im Gemisch noch kleinere Mengen, etwa bis maximal 10 Gew.-% des Gemisches an Laurinsäure, Myristinsäure, Pentadecansäure, Heptadecansäure, Ölsäure, Arachinsäure und/oder Behensäure enthalten sind. Diese Fettsäuren sind in technischen Fettsäurequalitäten übliche Begleitstoffe.

Die erfindungsgemäßen O/W-Emulsionen enthalten die Ölphase (O) und die wäßrige Phase (W) bevorzugt in einem Gewichtsverhältnis von O : W = 1 : 9 bis 4 : 6. Das Palmitinsäure-Stearinsäure-Gemisch ist bevorzugt in einer Menge von 1 bis 50 Gew.-%, bezogen auf die Ölphase, enthalten.

In den erfindungsgemäßen O/W-Emulsionen stellt die Seife eine Natrium-, Kalium-, Ammonium-, Mono-, Di- oder Trialkanolammonium-Seife mit 2 bis 4 C-Atomen in der Alkanolgruppe, bevorzugt eine Triethanolammonium-Seife dar. Es ist dabei nicht erforderlich, das Palmitinsäure-Stearinsäure-Gemisch vollständig in die Seife zu überführen. Bevorzugt wird nur ein Anteil des Fettsäuregemisches in die Seife überführt, der so bemessen ist, daß einerseits eine ausreichende Emulgierung erfolgt, andererseits der pH-Wert der Emulsion zwischen 4,5 und 8,5 liegt. Der nicht verseifte Anteil des Palmitinsäure-Stearinsäure-Gemisches stellt einen Anteil der zu emulgierenden Öl- und Fettkomponenten dar.

Die diskontinuierliche Ölphase besteht im wesentlichen aus kosmetischen oder pharmazeutischen Ölkomponenten, Fetten und/oder Wachsen, öllöslichen Emulgatoren, zu welchen auch die Palmitinsäure-Stearinsäure-Seifen gezählt werden und gegebenenfalls öllöslichen pharmazeutischen oder kosmetischen Wirkstoffen.

Als kosmetische Ölkomponenten eignen sich alle hierfür bekannten pflanzlichen, tierischen, mineralischen und synthetischen Öle. Als Beispiele seien genannt: Olivenöl, Sonnenblumenöl, Maiskeimöl, Spermöl, Nerzöl, Paraffinöl, Silikonöle, (z. B. Dimethylpolysiloxan) Squalan, Oleylalkohol, 2-Octyl-dodecanol, Decyloleat, Isopropylmyristat, Isononyl-stearat, 2-Ethylhexyl-palmitat, Glycerin-tricaprylat und andere als kosmetische Ölkomponenten bekannte Ester, Alkohole oder Kohlenwasserstoffe.

Eine bevorzugt geeignete kosmetische Ölkomponente ist das 2-Octyl-dodecanol, welches mit der erfindungsgemäß zu verwendenden Seife des Palmitinsäure-Stearinsäure-Gemisches besonders stabile Emulsionen ergibt. Das 2-Octyl-dodecanol wird bevorzugt in einer Menge von 10 - 50 Gew.-%, bezogen auf die Ölphase eingesetzt.

Als kosmetische Fette und Wachse eignen sich alle hierfür bekannten Produkte mit Schmelzpunkten bis ca. 80 °C, als Beispiele seien genannt gehärtete pflanzliche und tierische Fette (Triglyceride), Walrat, Fettalkohole, z. B. Cetylalkohol, Stearylalkohol, Ester wie z. B. Cetylpalmitat und natürliche Wachse wie z. B. Wollwachs, Bienenwachs, Japanwachs, Carnaubawachs, Candelillawachs, mineralische Wachse wie z. B. Montanwachs, Paraffine, Vaseline® sowie synthetische Paraffine wie z. B. die Polyethylenwachse. Hierzu gehören auch die gegebenenfalls enthaltenen unverseiften Anteile des Palmitinsäure-Stearinsäure-Gemisches. Der Anteil an solchen Wachsen ist im allgemeinen nicht höher als 50 Gew.-% der Ölphase. Als Emulgator wird bevorzugt die Seife des Palmitinsäure-Stearinsäure-Gemisches allein verwendet. Es können jedoch in untergeordneten Mengen, etwa bis 25 Gew.-% der gesamten Ölphase, auch andere, bekannte öllösliche O/W-Emulgatoren eingesetzt werden.

Als gegebenenfalls zusätzlich enthaltene öllösliche Emulgatoren eignen sich alle für die Emulgierung der vorgenannten Öle, Fette und Wachse geeigneten, öllöslichen Emulgatoren. Als Beispiele seien genannt die Seifen von Fettsäuren mit 12 bis 15 oder mit 20 bis 22 C-Atomen, die Mono- und Diglyceride und die Sorbitan-Partialester von Fettsäuren mit 12 - 22 C-Atomen und die Anlagerungsprodukte von 2 - 30 Mol Ethylenoxid an solche Fettsäure-Partialglyceride und Sorbitanfettsäureester, die Anlagerungsprodukte von 2 - 30 Mol Ethylenoxid an Fettalkohole mit 12 - 22 C-Atomen, an Fettsäuren mit 12 - 22 C-Atomen, an Alkylphenole mit 8 - 16 C-Atomen in der Alkylgruppe und an Fettsäurealkanolamide, die Fettalkoholsulfate mit 16 - 22 C-Atomen in Form ihrer Alkali- oder Alkanolammoniumsalze, die Phosphorsäureester linearer Fettalkohole oder von Fettalkoholpolyglycolethern in Form der Alkali- oder Alkanolammoniumsalze.

Neben den genannten Bestandteilen kann die diskontinuierliche Ölphase noch öllösliche Wirkstoffe wie z. B. Lichtschutzmittel, Antioxydantien, Vitamine, öllösliche Konservierungsmittel, z. B. p-Hydroxybenzoesäurebenzylester oder pharmazeutische Wirkstoffe enthalten.

Die kontinuierliche wäßrige Phase der erfindungsgemäßen O/W-Emulsionen kann im einfachsten Falle aus Wasser und Alkalisierungsmittel (wie z. B. Kaliumhydroxid, Triethanolamin) bestehen. Bevorzugt werden aber der wäßrigen Phase der Emulsion Hilfsmittel zugesetzt, die die Stabilität und die Anwendungseigenschaften günstig beeinflussen. Bei kosmetischen O/W-Emulsionen, die der Pflege der Haut und/oder des Haares dienen, empfiehlt es sich, in die wäßrige Phase zur Kältestabilisierung 1 bis 30 Gew.-%, bezogen auf die Wasserphase, eines Glycols oder Polyols aus der Gruppe Propylenglycol, Polyethylenglycol, Hexylenglycol, Glycerin und/oder Sorbit und 0,05 bis 3,0 Gew.-%, bezogen auf die Wasserphase, eines wasserlöslichen Polymeren aus der Gruppe der wasserlöslichen Polysaccharide, der wasserlöslichen Polysaccharidether, der Acrylsäure-Polymerisate und-Copolymerisate, Polyvinylalkohol und/oder Polyvinylpyrrolidon oder einer Mischung solcher Polymeren zuzugeben.

Besonders vorteilhaft ist die Zugabe einer Kombination aus (a) einem wasserlöslichen nichtionischen Celluloseether und (b) einem vernetzten Acrylsäure-Polymerisat oder Copolymerisat mit einem mittleren Molekulargewicht von 1 000 000 bis 5 000 000 in Form eines wasserlöslichen Salzes im Mengenverhältnis (a) : (b) = 9 : 1 bis 1 : 9. Durch diese Kombination von Hydrocolloiden wird die Viskosität der wäßrigen Phase synergistisch gesteigert, so daß die auf diese Weise erhaltenen O/W-Emulsionen beim Verteilen auf der Haut mehr "Körper" zeigen.

Als vernetzte Acrylsäure-Polymerisate eignen sich Produkte, die durch Copolymerisation von Acrylsäure mit 0,1 - 4,0 Gew.-% eines Polyalkenyl-polyethers eines mehrwertigen Alkohols mit mehr als einer Alkenylethergruppe im Molekül als Vernetzungsmittel erhalten werden. Ein Beispiel für ein solches Vernetzungsmittel ist z. B. Polyallylsucrose.

Gegebenenfalls können bei der Herstellung der vernetzten Acrylsäure-Polymerisate auch weitere Comonomere in Mengen bis zu 59 Gew.-% der Monomerenmischung eingesetzt werden. Geeignete Comonomere sind z. B. Maleinsäureanhydrid, N-Methylacrylamid, Methyl-vinylether oder Mischungen solcher zusätzlicher Monomerer. Solche vernetzte Acrylsäurepolymerisate sind z. B. aus US-PS 2.798.053 bekannt und unter dem Warenzeichen CARBOPOL (R) (der Fa. Goodrich) im Handel. Die vernetzten Acrylsäure-Polymerisate lassen sich in Wasser dispergieren, die starke Verdickungswirkung wird jedoch erst erreicht, wenn die Polymerisate durch anorganische Basen wie z. B. Natriumhydroxid, Kaliumhydroxid, Ammoniak oder durch niedermolekulare Amine oder Alkanolamine in die Salzform überführt werden.

Die kontinuierliche wäßrige Phase kann neben den genannten Glycolen oder Polyolen und wasserlöslichen Polymeren weitere wasserlösliche Hilfsmittel enthalten. Als solche können z. B. wasserlösliche Salze, z. B. Puffersubstanzen, z. B. Alkaliphosphat, Alkalicitrat, Borate, wasserlösliche Konservierungsmittel wie z. B. p-Hydroxybenzoesäuremethylester, Sorbinsäure, wasserlösliche oberflächenaktive Stoffe oder Emulgatoren, wasserlösliche Farbstoffe oder wasserlösliche kosmetische oder pharmazeutische Wirkstoffe, z. B. wasserlösliche Pflanzenextrakte, wasserlösliche Proteine oder Proteinderivate, Aminosäuren usw. genannt werden.

Die Herstellung der erfindungsgemäßen O/W-Emulsion erfolgt zweckmäßigerweise wie folgt:

Die Ölkomponenten, Fettstoffe und gegebenenfalls Wachse werden mit dem Palmitinsäure-Stearinsäure-Gemisch und gegebenenfalls mit weiteren öllöslichen Emulgatoren und öllöslichen Wirkstoffen gemischt und bis zum Erreichen einer homogenen Schmelze erhitzt. Die Komponenten der wäßrigen Phase, d. h. das Wasser, die bevorzugt enthaltenen Glycole oder Polyole, die wasserlöslichen Polymeren, die gegebenenfalls enthaltenen weiteren wasserlöslichen Hilfsmittel und die für die Verseifung des Palmitinsäure-Stearinsäure-Gemisches erforderliche Menge an Base, z. B. Natronlauge, Kalilauge, Ammoniaklösung oder z. B. Triethanolamin werden im Wasser gelöst und auf ca. 70 bis 90 °C erwärmt. Anschließend werden die Ölphase und die wäßrige Phase unter intensivem Rühren miteinander vermischt und für die Neutralisation mindestens 5 Minuten bei 70 bis 90 °C gerührt. Die dabei entstehende Emulsion wird auf Raumtemperatur abgekühlt.

Die erfindungsgemäßen Emulsionen zeichnen sich durch eine besondere Feinteiligkeit und Stabilität und auch durch eine Viskosität aus, die merklich höher ist als die von Emulsionen sonst gleicher Zusammensetzung, die mit der Seife von Palmitinsäure, Stearinsäure oder eines Palmitinsäure-Stearinsäure-Gemisches mit weniger als 60 Gew.-% Palmitinsäure oder mit weniger als 20 Gew.-% Stearinsäure hergestellt wurde.

Die folgenden Beispiele sollen den Erfindungsgegenstand näher erläutern, ohne ihn hierauf zu beschränken:

Beispiele

# B e i s p i e l e

| Hautemulsionen | Nr. | 1 | 2 | 3 |
|---|---|---|---|---|
| Paraffinöl, dünnflüssig (paraffinum perliquidum DAB) | | 15 | 12 | 10 |
| Isopropylpalmitat | | – | 3 | 5 |
| Palmitinsäure-Stearinsäure-Gemisch (70 Gew.-% $C_{16}$, 30 Gew.-% $C_{18}$ | | 0,5 | 0,5 | 0,5 |
| Propylenglycol | | 3 | 3 | 3 |
| Triethanolamin | | 0,2 | 0,2 | 0,2 |
| p-Hydroxybenzolsäuremethylester | | 0,2 | 0,2 | 0,2 |
| p-Hydroxybenzolsäurepropylester | | 0,1 | 0,1 | 0,1 |
| Carboxyvinylpolymerisat (Carbopol ® 940) | | 0,1 | 0,15 | 0,15 |
| Wasser | | ad 100 | ad 100 | ad 100 |

Die Hautemulsion gemäß Beispiel Nr. 1 wurde bei sonst gleicher Zusammensetzung mit Palmitinsäure-Stearinsäure-Gemischen hergestellt, deren $C_{16}/C_{18}$-Gewichtsverhältnis von 100/0 bis 0/100 variiert wurde.

Es wurden in allen Fällen O/W-Emulsionen erhalten, die jedoch bezüglich Aussehen (Feinteiligkeit), Lagerstabilität bei 50 °C und Viskosität Unterschiede aufwiesen.

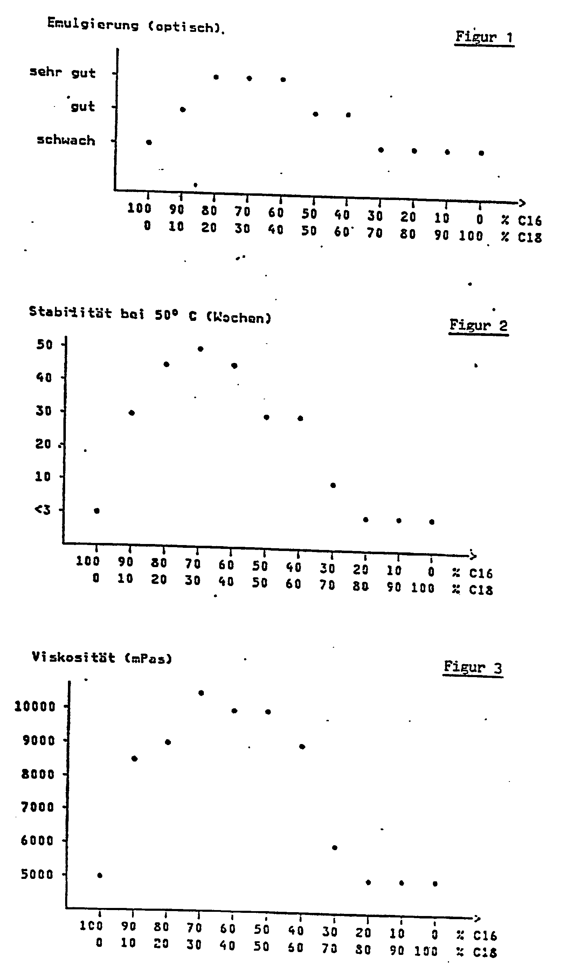

Aus den folgenden Diagrammen ist ersichtlich, daß die Emulsionen auf Basis von Palmitinsäure-Stearinsäure-Gemischen mit 60 bis 80 Gew.-% Palmitinsäure und 20 bis 40 Gew.-% Stearinsäure, insbesondere mit 70 Gew.-% Palmitinsäure und 30 Gew.-% Stearinsäure
- ein signifikant besseres, feinteiligeres Aussehen (Figur 1),

- eine deutlich längere Lagerstabilität bei 50 °C (Figur 2) und
- eine höhere Viskosität (Figur 3) aufweisen.

Erläuterungen zu den Figuren:

Figur 1: Die Beurteilung des Aussehens erfolgte sofort nach der Herstellung nach den folgenden Kriterien:

sehr gut: sehr feinteilige, weiße Emulsion gut: weniger feinteilige, weiße Emulsion schwach: grobe Tröpfchen, opake Emulsion

Figur 2: Die Proben wurden in verschlossenen Gefäßen bei 50 °C im Thermostat so lange gelagert, bis deutlich sichtbare Inhomogenität oder Separation auftrat.

Figur 3: Die Messung der Viskosität erfolgte 48 Stunden nach der Herstellung bei 20 °C mit einem Rotationsviskosimeter, Typ Brookfield, Spindel 5 bei 10 UpM.

EP 0 214 568 B1

Weitere Anwendungsbeispiele:

4. Pflegecreme, O/W

| | | |
|---|---|---|
| Cutina (R) MD [1] | 5,0 | Gew.-% |
| 2-Octyl-dodecanol | 4,0 | " |
| Paraffinöl, dünnflüssig | 4,0 | " |
| Palmitin-Stearinsäure (70 : 30) | 3,0 | " |
| Cetylalkohol | 2,0 | " |
| Glycerin, 86gew.-%ig | 3,0 | " |
| Kaliumhydroxid | 0,25 | " |
| Carbopol (R) 934 [9] | 0,2 | " |
| Wasser | ad 100,0 | " |

5. Nachtcreme, O/W

| | | |
|---|---|---|
| Palmitin-Stearinsäure (70 : 30) | 6,0 | Gew.-% |
| Cutina (R) MD [1] | 5,0 | " |
| Cetylalkohol | 1,0 | " |
| Eumulgin (R) B1 [2] | 1,0 | " |
| 2-Octyl-dodecanol | 8,0 | " |
| Myritol (R) 318 [4] | 3,0 | " |
| Paraffinöl, dünnflüssig | 3,0 | " |
| Hydagen (R) F [5] | 0,2 | " |
| Carbopol (R) 940 [8] | 0,4 | " |
| Triethanolamin | 1,5 | " |
| Wasser | ad 100,0 | " |

6. Feuchtigkeitsemulsion, O/W

| | | |
|---|---|---|
| Palmitin-Stearinsäure (70 : 30) | 4,0 | Gew.-% |
| Cetyl-Stearylalkohol | 2,0 | " |
| Eumulgin (R) B2 [3] | 2,0 | " |
| Isopropylmyristat | 5,0 | " |
| 2-Octyldodecanol | 2,0 | " |
| Myritol (R) 318 [4] | 3,0 | " |
| Sorbit, 70%ig | 3,0 | " |
| Carbopol (R) 941 [10] | 0,7 | " |
| Viscontran (R) MHPC 3000 [6] | 0,6 | " |
| Triethanolamin | 1,5 | " |
| Wasser | ad 100,0 | " |

6

## 7. Pflegemilch, O/W

| | |
|---|---|
| Palmitin-Stearinsäure (70 : 30) | 2,5 Gew.-% |
| Paraffinöl, dünnflüssig | 4,0 " |
| Hostaphat (R) KW 340 N 7) | 2,0 " |
| 2-Octyl-dodecanol | 8,0 " |
| Cetyl-Stearylalkohol | 1,0 " |
| Glycerin, 99,5%ig | 5,0 " |
| Triethanolamin | 0,9 " |
| Pflanzenextrakte, wäßrig | 10,0 " |
| Carbopol (R) 940 8) | 0,2 " |
| Viscontran (R) MHPC 3000 6) | 0,3 " |
| Wasser | ad 100,0 " |

## 8. Nachtcreme, O/W

| | |
|---|---|
| Palmitin-Stearinsäure (65 : 35) | 10,0 Gew.-% |
| Cutina (R) MD 1) | 2,0 " |
| Eumulgin (R) B1 2) | 1,0 " |
| Brij 52 11) | 1,0 " |
| 2-Octyl-dodecanol | 2,0 " |
| Paraffinöl, dünnflüssig | 8,0 " |
| Mandelöl | 3,0 " |
| Triethanolamin | 2,0 " |
| Glycerin, 86 Gew.-% | 4,0 " |
| Wasser | ad 100,0 " |

In den Rezepturen 4 bis 8 wurden folgende Warenzeichen verwendet:

1) Cutina (R) MD: Gemisch von Mono- und Diglyceriden der Palmitin- und Stearinsäure (Henkel KGaA)
2) Eumulgin (R) B1: Cetyl-stearylalkohol + 12 Mol Ethylenoxid (Henkel KGaA)
3) Eumulgin (R) B2: Cetyl-stearylalkohol + 20 Mol Ethylenoxid (Henkel KGaA)
4) Myritol (R) 318: Capryl-caprinsäure-triglycerid (Henkel KGaA)
5) Hydagen (R) F: Natriumsalz einer Polyhydroxycarbonsäure (Henkel KGaA)
6) Viscontran (R)
MHPC 3000: Methylhydroxypropylcellulose, Viskosität 2%ig in destilliertem Wasser (20 °C, Brookfield-Viskosimeter, 20 UpM) 2 900 - 4 400 mPas (Henkel KGaA)
7) Hostaphat
KW 340 N: Phosphorsäureester eines Wachsalkohol + 4 Mol Ethylenoxid-Addukts (Hoechst AG)
8) Carbopol (R) 940: Vernetztes Acrylsäure-Polymerisat, mittleres Molekulargewicht ca. 4 000 000 (B. F. Goodrich Chem. Comp. Avon Lake, Ohio, USA)
9) Carbopol (R) 934: Vernetztes Acrylsäure-Polymerisat, mittleres Molekulargewicht ca. 3 000 000 (B. F. Goodrich Chem. Comp.)

10) Carbopol (R) 941: Vernetztes Acrylsäure-Polymerisat, mittleres Molekulargewicht ca. 1 250 000 (B. F. Goodrich Chem. Comp.)

11) Brij 52: Cetylalkohol + 2 Mol Ethylenoxid (ICI)

## Patentansprüche

1. Öl-in-Wasser-Emulsion, bestehend aus einer kontinuierlichen wäßrigen Phase und einer diskontinuierlichen Ölphase, mit einem Emulgator, der ganz oder überwiegend aus der Seife eines Palmitinsäure-Stearinsäure-Gemisches besteht, dadurch gekennzeichnet, daß das Palmitinsäure-Stearinsäure-Gemisch 60 bis 80 Gew.-% Palmitinsäure und 20 bis 40 Gew.-% Stearinsäure enthält.

2. Öl-in-Wasser-Emulsion nach Anspruch 1, dadurch gekennzeichnet, daß die Seife eine Natrium-, Kalium-, Ammonium-, Mono-, Di- oder Trialkanolammonium-Seife mit 2 bis 4 C-Atomen in der Alkanolgruppe darstellt.

3. Öl-in-Wasser-Emulsion nach den Ansprüchen 1 oder 2, dadurch gekennzeichnet, daß das Palmitinsäure-Stearinsäure-Gemisch aus ca. 70 Gew.-% Palmitinsäure und ca. 30 Gew.-% Stearinsäure besteht.

4. Öl-in-Wasser-Emulsion nach den Ansprüchen 1 bis 3, dadurch gekennzeichnet, daß die Ölphase (O) und die wäßrige Phase (W) in einem Gewichtsverhältnis von O : W = 1 : 9 bis 4 : 6 enthalten sind, das Palmitinsäure-Stearinsäure-Gemisch in einer Menge von 1 bis 50 Gew.-%, bezogen auf die Ölphase, enthalten ist und nur so weit verseift ist, daß die Emulsion einen pH-Wert zwischen 4,5 und 8,5 aufweist.

5. Öl-in-Wasser-Emulsion nach den Ansprüchen 1 bis 4, dadurch gekennzeichnet, daß die Ölphase 2-Octyl-dodecanol als kosmetische Ölkomponente in einer Menge von 10 bis 50 Gew.-%, bezogen auf die Ölphase enthält.

6. Öl-in-Wasser-Emulsion nach den Ansprüchen 1 bis 5, dadurch gekennzeichnet, daß die wäßrige Phase 1 bis 30 Gew.-% eines Glycols oder Polyols aus der Gruppe Propylenglycole, Polyethylenglycole, Hexylenglycol, Glycerin und/oder Sorbit und 0,05 bis 3 Gew.-% eines wasserlöslichen Polymeren aus der Gruppe der wasserlöslichen Polysaccharide, der wasserlöslichen Polysaccharidether, der gegebenenfalls vernetzten Acrylsäure-Polymerisate und -Copolymerisate, Polyvinylalkohol und/oder Polyvinylpyrroliden, bezogen auf das Gewicht der wäßrigen Phase, enthält.

7. Verwendung eines Palmitinsäure-Stearinsäure-Gemisches, bestehend aus 60 bis 80 Gew.-% Palmitinsäure und 20 bis 40 Gew.-% Stearinsäure zur Herstellung von Wasser-in-Öl-Emulsionen nach den Ansprüchen 1 bis 6.

## Claims

1. An oil-in-water emulsion consisting of a continuous aqueous phase and a discontinuous oil phase and containing an emulsifier which consists entirely or predominantly of the soap of a palmitic acid-stearic acid mixture, characterized in that the palmitic acid-stearic acid mixture contains 60 to 80% by weight palmitic acid and 20 to 40% by weight stearic acid.

2. An oil-in-water emulsion as claimed in claim 1, characterized in that the soap is a sodium, potassium, ammonium, mono-, di-, or trialkanolammonium soap containing from 2 to 4 C-atoms in the alkanol group.

3. An oil-in-water emulsion as claimed in claim 1 or 2, characterized in that the palmitic acid-stearic acid mixture consists of approximately 70% by weight palmitic acid and approximately 30% by weight stearic acid.

4. An oil-in-water emulsion as claimed in claims 1 to 3, characterized in that the oil phase (o) and the aqueous phase (w) are present in a ratio by weight of from 1 : 9 to 4 : 6 o : w, the palmitic acid-stearic acid mixture is present in a quantity of from 1 to 50% by weight, based on the oil phase, and is only saponified to the extent that the emulsion has a pH value of from 4.5 to 8.5.

5. An oil-in-water emulsion as claimed in claims 1 to 4, characterized in that the oil phase contains 2-octyl dodecanol as cosmetic oil component in a quantity of 10 to 50% by weight, based on the oil phase.

6. An oil-in-water emulsion as claimed in claims 1 to 5, characterized in that the aqueous phase contains from 1 to 30% by weight of a glycol or polyol from the group comprising propylene glycols, polyethylene glycols, hexylene glycol, glycerol and/or sorbitol and from 0.05 to 3% by weight of a water-soluble polymer from the group comprising water-soluble polysaccharides, water-soluble polysaccharide ethers, optionally crosslinked acrylic acid polymers and copolymers, polyvinyl alcohol and/or polyvinyl pyrrolidone based on the weight of the aqueous phase.

7. The use of a palmitic acid-stearic acid mixture consisting of from 60 to 80% by weight palmitic acid and from 20 to 40% by weight stearic acid for preparing the water-in-oil emulsions claimed in claims 1 to 6.

## Revendications

1. Emulsion d'huile dans l'eau se composant d'une phase aqueuse continue et d'une phase huileuse discontinue, et comportant un émulsionnant qui se compose, en totalité ou d'une manière prédominante, d'un savon d'un mélange acide palmitique/acide stéarique, caractérisée en ce que le mélange acide palmitique/acide stéarique contient de 60 à 80% en poids d'acide palmitique et de 20 à 40% en poids d'acide stéarique.

2. Emulsion d'huile dans l'eau selon la revendication 2, caractérisée en ce que le savon correspond à un savon de sodium, de potassium, d'ammonium, de mono-, de di- ou de tri-alcanolammonium ayant de 2 à 4 atomes de C dans le groupe alcanol.

3. Emulsion d'huile dans l'eau selon les revendications 1 ou 2, caractérisée en ce que le mélange acide palmitique/acide stéarique se compose d'environ 70% en poids d'acide palmitique et d'environ 30% en poids d'acide stéarique.

4. Emulsion d'huile dans l'eau selon les revendications 1 à 3, caractérisée en ce que la phase huileuse (H) et la phase aqueuse (E) sont présentes dans un rapport pondéral H/E = 1 : 9 à 4 : 6, et en ce que le mélange acide palmitique/acide stéarique est présent en une quantité allant de 1 à 50% en poids rapporté à la phase huileuse, et n'est saponifié que dans une mesure suffisante pour que l'émulsion possède une valeur de pH comprise entre 4,5 et 8,5.

5. Emulsion d'huile dans l'eau selon les revendications 1 à 4, caractérisée en ce que la phase huileuse contient comme composant huileux cosmétique du 2-octyl-dodécanol en une quantité allant de 10 à 50% en poids rapporté à la phase huileuse.

6. Emulsion d'huile selon les revendications 1 à 5, caractérisée en ce que la phase aqueuse contient de 1 à 30% en poids d'un glycol ou d'un polyol choisi dans le groupe formé par les propylèneglycols, polyéthylèneglycols, hexylèneglycol glycérol et/ou sorbitol et 0,05 à 3% en poids d'un polymère soluble dans l'eau choisi dans le groupe constitué des polysaccharides hydrosolubles, éthers de polysaccharides hydrosolubles, polymères et copolymères d'acide acrylique éventuellement réticulés, alcool polyvinylique et/ou polyvinylpyrrolidone, rapporté au poids de la phase aqueuse.

7. Utilisation d'un mélange acide palmitique/acide stéarique qui se compose de 60 à 80% en poids d'acide palmitique et de 20 à 40% en poids d'acide stéarique pour l'obtention d'émulsions d'huile dans l'eau selon les revendications 1 à 6.

Emulgierung (optisch).

Figur 1

Stabilität bei 50° C (Wochen)

Figur 2

Viskosität (mPas)

...